(19) Europäisches Patentamt
European Patent Office
Office européen des brevets

(11) Veröffentlichungsnummer: **0 027 215**
**B1**

(12) # EUROPÄISCHE PATENTSCHRIFT

(45) Veröffentlichungstag der Patentschrift: **13.04.83**

(51) Int. Cl.³: **A 61 B 5/02**, A 61 B 10/00

(21) Anmeldenummer: **80105974.2**

(22) Anmeldetag: **02.10.80**

(54) **Kardiotachometer.**

(30) Priorität: **15.10.79 DE 2941668**

(43) Veröffentlichungstag der Anmeldung: **22.04.81 Patentblatt 81/16**

(45) Bekanntmachung des Hinweises auf die Patenterteilung: **13.04.83 Patentblatt 83/15**

(84) Benannte Vertragsstaaten: **DE FR GB IT**

(56) Entgegenhaltungen:
**AT-B-297 207**
**DE-A-1 905 620**
**DE-A-2 304 173**
**DE-B-2 219 045**
**US-A-3 498 290**
**US-A-3 561 430**
**US-A-3 676 584**
**US-A-3 780 725**

(73) Patentinhaber: **Fraunhofer-Gesellschaft zur Förderung der angewandten Forschung e.V., Leonrodstrasse 54, D-8000 München 19 (DE)**

(72) Erfinder: **Mauser, Rudolf, Haussmannstrasse 146B, D-7000 Stuttgart 1 (DE)**
Erfinder: **Chmiel, Horst, Prof-Dr., Paracelsus-Strasse 14, D-7250 Leonberg-Ramtel (DE)**

(74) Vertreter: **Kraus, Walter, Dr. et al, Patentanwälte Dres. Kraus & Weisert Irmgardstrasse 15, D-8000 München 71 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 027 215 B1

BUNDESDRUCKEREI BERLIN

Kardiotachometer

Die Erfindung betrifft ein Kardiotachometer zum Ermitteln der Herzfrequenz unter Berücksichtigung von ventrikulären Extrasystolen mittels einer nach dem Ultraschall-Doppler-Prinzip arbeitenden Ultraschall-Sende- und -Empfangseinrichtung mit nachgeschalteter Signalverarbeitungs-Schaltungsanordnung zum Feststellen einer Gefäßwandbewegung und einer weiteren Ultraschall-Sende- und -Empfangseinrichtung mit nachgeschalteter weiterer Signalverarbeitungs-Schaltungsanordnung zum Ermitteln der Blutströmungsgeschwindigkeit sowie einer beiden Ultraschall-Sende- und -Empfangseinrichtungen und deren Signalverarbeitungs-Schaltungsanordnungen nachgeschalteten Signalkorrelierungseinrichtung.

Kardiotachometer zum Ermitteln der Herzfrequenz mittels einer nach dem Ultraschall-Doppler-Prinzip arbeitenden Ultraschall-Sende- und -Empfangseinrichtung mit nachgeschalteter Signalverarbeitungs-Schaltungsanordnung zum Feststellen einer Gefäßwandbewegung sind beispielsweise aus den DE-A-2 219 045 und 2 331 551 bekannt. Mit diesen Herzfrequenzmeßgeräten wird Ultraschall direkt auf die Herzwand aufgestrahlt, so daß also als Gefäßwandbewegung die Bewegung der Herzwand direkt ermittelt wird. Das geschieht nach der DE-A-2 219 045 dadurch, daß der darin vorgeschlagene Herzfrequenzmesser ein seinem Ultraschallempfänger nachgeschaltetes Selektionsglied hat, welches lediglich Empfangssignale, die aus einer der beiden Herzpulsationsbewegungen stammen, nämlich entweder aus der zum Ultraschallsystem hin oder aus der vom Ultraschallsystem weg erfolgenden Herzpulsationsbewegung, zu einem Dopplerdiskriminator durchläßt. Diesem Dopplerdiskriminator ist ein Glied zur Unterdrückung von Dopplerkomponenten mit einer Frequenz, die höher als die höchste zu erwartende Frequenz der von der Herzwandbewegung stammenden Dopplerkomponenten ist, nachgeschaltet, auf den ein Amplitudenmodulator zur Amplitudenmodulation der frequenzbegrenzten Dopplersignale, ein Glied zur Unterdrückung von Frequenzen des Demodulationssignals, die größer als die höchste zu erwartende Herzfrequenz sind, eine Differenziereinrichtung zum Differenzieren des frequenzbegrenzten Demodulationssignals sowie ein Impulserzeuger folgen; letzterer gibt einen Spannungsimpuls ab, wenn das von der Differenziereinrichtung abgegebene Signal einen vorbestimmten Schwellenwert überschreitet.

Die Herzfrequenzmessung nach der weiterhin genannten DE-A-2 331 551 geschieht dagegen in der Weise, daß die mit einer Ultraschall-Sende- und -Empfangseinrichtung gewonnenen Dopplersignale einer Ausleseeinheit zugeführt werden, in welcher je drei aufeinanderfolgende Signale verglichen werden, um ein Kriterium zur Unterscheidung zwischen Nutzsignalen, d. h. Signalen, die einem Herzschlag entsprechen, und Störsignalen festzulegen. Die Ausleseeinheit steuert eine Torschaltung, über die die Nutzsignale an eine Ausgangseinheit abgegeben werden, während die Störsignale von dieser Torschaltung nicht durchgelassen werden. Die Ausgangseinheit bildet einen Mittelwert der Nutzsignale über ein Intervall von drei Herzschlägen, um ein Ausgangssignal zu gewinnen, das der Herzfrequenz entspricht.

In den beiden Herzfrequenzmeßgeräten nach den DE-A-2 219 045 und 2 331 551 werden zwar gewisse Störungen ausgeschieden, aber diese Herzfrequenzmesser unterscheiden nicht zwischen Systolen und Extrasystolen des Herzens, und sie ermöglichen auch nicht eine gesonderte Anzeige der normalen Herzfrequenz, d. h., der Häufigkeit der Systolen einerseits und eine Anzeige der Häufigkeit von ventrikulären Extrasystolen andererseits.

Aus der US-A-3 498 290 ist eine nach dem Ultraschall-Doppler-Prinzip arbeitende erste Ultraschall-Sende- und -Empfangseinrichtung mit nachgeschalteter Signalverarbeitungs-Schaltungsanordnung zum Feststellen des Abstandes zweier Gefäßwände, nämlich des Durchmessers eines Blutgefäßes, bekannt. Hierbei ist die Zeitdifferenz zwischen je zwei empfangenen Doppler-Reflexionen proportional dem Durchmesser, und es können auch kleine Veränderungen des Blutgefäßdurchmessers berücksichtigt werden. Diese Einrichtung ist insofern gleichwertig mit einer Einrichtung zum Feststellen einer Gefäßwandbewegung, als sich die erwähnte Zeitdifferenz proportional mit den Veränderungen des Durchmessers ändert.

Wenn es auch grundsätzlich möglich wäre, mit der Einrichtung nach der US-A-3 498 290 durch viele, schnell hintereinander erfolgende fortlaufende Durchmesserberechnungen den zeitlichen Verlauf der Gefäßwandbewegung zu ermitteln, so wäre das doch sehr aufwendig.

Es ist jedoch aus der DE-A-1 905 620 bekannt, mittels des Ultraschall-Doppler-Prinzips auch auf einfachere Weise eine Gefäßwandbewegung eines Blutgefäßes festzustellen. Hier wird die Geschwindigkeit der Gefäßwandbewegung senkrecht zur Längsausdehnung des Blutgefäßes an zwei in Längsabstand voneinander befindlichen Stellen gemessen und daraus die Pulswellengeschwindigkeit berechnet.

In der Einrichtung nach der US-A-3 498 290 sind außerdem noch eine weitere Ultraschall-Sende- und -Empfangseinrichtung mit nachgeschalteter weiterer Signalverarbeitungs-Schaltanordnung zum Ermitteln der Blutströmungsgeschwindigkeit sowie eine den beiden Ultraschall-Sende- und -Empfangseinrichtungen nachgeschaltete Signalkorrelierungseinrichtung zum Feststellen des Durchflußvolumens unter Berücksichtigung der Pulsfrequenz bzw. unter Berücksichtigung der Herzfrequenz vorgesehen, so daß insgesamt aus der US-A-3 498 290 eine Einrichtung der eingangs genannten Art bekannt ist, die es jedoch ebenso wie die Einrichtung nach der DE-A-1 905 620 nicht ermöglicht, die Herzfrequenz unter Berücksichtigung von ventrikulären Extrasystolen zu ermitteln.

Die Berücksichtigung von ventrikulären Extrasystolen ist jedoch sehr wichtig, denn beim Auftreten von ventrikulären Extrasystolen kommt es nämlich aufgrund der vorzeitigen Kontraktion des Herzmuskels je nach Vorzeitigkeitsindex zu einem mehr oder weniger stark verminderten Blutauswurf des Herzens. Dies hat seine Ursache unter anderem darin, daß durch die zu kurze Füllungszeit nicht genügend Blut in den Ventrikel zurückströmen konnte. Die Ursache bzw. die Entstehungsart von solchen ventrikulären Extrasystolen kann verschiedener Natur sein, wobei derartige ventrikuläre Extrasystolen jedoch nie aus dem normalen Reizleitungssystem hervorgehen, sondern, wie schon der Name sagt, aus den Ventrikeln. Treten solche ventrikulären Extrasystolen gehäuft und/oder sehr früh nach einer Systole der normalen Herztätigkeit auf, dann besteht die Gefahr eines Herzflimmerns, das tödlich ausgeht, wenn nicht augenblicklich eine Reanimation und Defibrilation erfolgt. Wie man hieraus ersieht, ist die gesonderte Erfassung der Häufigkeit der ventrikulären Extrasystolen von erheblicher medizinischer Bedeutung.

Auch die normale, von einer Verfälschung durch Extrasystolen bereinigte Herzfrequenz, also die reine Systolen-Häufigkeit, ist medizinisch sehr wichtig. Diese Herzfrequenz sollte nämlich stets innerhalb eines bestimmten Bereiches liegen, der individuell je nach der körperlichen Konstitution und dem Gesundheitszustand der jeweiligen Person verschieden ist.

Aufgabe der Erfindung ist es daher, ein Kardiotachometer der eingangs genannten Art so auszubilden, daß es bei relativ einfacher Ausbildung eine Unterscheidung zwischen Systolen und ventrikulären Extrasystolen ermöglicht, damit die mittlere zeitliche Häufigkeit der Systolen und der Extrasystolen gesondert gemessen werden kann.

Diese Aufgabe wird erfindungsgemäß dadurch gelöst, daß die Signalkorrelierungseinrichtung eine Vergleichseinrichtung zum Feststellen einer zeitlichen Koinzidenz von Ausgangssignalen der beiden Ultraschall-Sende- und -Empfangseinrichtungen mit dem Zweck einer Unterscheidung zwischen Systolen und ventrikulären Extrasystolen ist.

Ein solches Kardiotachometer ermöglicht eine Erfassung von Systolen und ventrikulären Extrasystolen des Herzens bei Unterscheidung zwischen diesen beiden Arten, indem die erwähnten Dopplerfrequenzmessungen nicht direkt am Herzen vorgenommen werden, sondern beispielsweise an einer Arterie, wie zum Beispiel der arteria radialis, wobei die Unterscheidung zwischen Systolen und ventrikulären Extrasystolen aufgrund der Tatsache erfolgt, daß die Systolen eine Pulswelle im Blutgefäß, beispielsweise in einer Arterie, erzeugen, die von einer deutlichen Wandbewegung dieses Blutgefäßes begleitet ist, während die ventrikulären Extrasystolen nur eine sogenannte Verschiebungswelle in dem Blutgefäß, beispielsweise in der Arterie, zur Folge haben, bei der keine merkliche Wandbewegung dieses Blutgefäßes auftritt. Wenn nun ein durch die Messung der Blutströmungsgeschwindigkeit erhaltenes impulsförmiges Doppler-Blutströmungsgeschwindigkeitsmeßsignal zeitlich mit einem durch die Ermittlung der Gefäßwandbewegung gewonnenen impulsförmigen Doppler-Gefäßwandbewegungssignal zusammenfällt, dann liegt eine Systole vor, während beim Fehlen eines gleichzeitigen Doppler-Gefäßwandbewegungssignals das Doppler-Blutströmungsgeschwindigkeitssignal einer ventrikulären Extrasystole entspricht. Diese Unterscheidung wird in der vorgenannten Vergleichseinrichtung durchgeführt, die vorzugsweise nur dann ein impulsförmiges Ausgangssignal, das einem Systolen-Zählimpuls entspricht, liefert, wenn gleichzeitig ein Doppler-Blutströmungsgeschwindigkeitssignal und ein Doppler-Gefäßwandbewegungssignal auftreten.

Zwar sind Vorrichtungen zur automatischen Erfassung vorzeitiger Extrasystolen an sich bekannt, wie zum Beispiel aus der DE-A-2 304 173, jedoch sind diese Vorrichtungen sehr aufwendig, da hiermit, wie aus der DE-A-2 304 173 ersichtlich ist, die Extrasystolen durch EKG-Messungen und deren Auswertung mittels Prozeßrechner erfaßt werden.

Das erfindungsgemäße Kardiotachometer ist vorzugsweise so ausgebildet, daß der Ultraschallsender der Ultraschall-Sende- und -Empfangseinrichtung zum Ermitteln der Blutströmungsgeschwindigkeit auf eine Ultraschallfrequenz im Bereich von 5 bis 10 MHz, vorzugsweise 5 MHz, abgestimmt ist, während der Ultraschallsender der Ultraschall-Sende- und -Empfangseinrichtung zum Ermitteln der Gefäßwandbewegung auf eine Ultraschallfrequenz im Bereich von 1 bis 3 MHz, vorzugsweise auf 1 MHz, abgestimmt ist. Die Ultraschallempfänger können in entsprechender Weise abgestimmt sein.

Durch diese beiden unterschiedlichen Frequenzen, von denen die eine wesentlich größer als die andere ist, können die Blutströmungsgeschwindigkeit und die Gefäßwandbewegung gesondert und in ausreichendem Umfang erfaßt werden.

Aus der bereits oben erwähnten DE-A-1 905 620 ist es zwar bekannt, für die Ermittlung der Gefäßwandgeschwindigkeit Ultraschallfrequenzen zwischen 2 MHz und 12 MHz zu verwenden. Jedoch arbeiten die beiden dort beschriebenen Ultraschallsender auf der gleichen Frequenz, da sie von einem gemeinsamen Oszillator betrieben werden.

Im einzelnen kann das Kardiotachometer nach der Erfindung schaltungsmäßig so ausgebildet sein, daß die Signalverarbeitungs-Schaltungsanordnung, welche der Ultraschall-Sende- und -Empfangseinrichtung zum Ermitteln der Blutströmungsgeschwindigkeit nachgeschaltet ist, eine erste Impulserzeugungseinrichtung, insbesondere eine Rechteckimpulserzeugungseinrichtung, ist, die auf die erhaltenen Ultraschall-Dopplerfrequenzsignale in der Weise anspricht, daß sie je einen Impuls für jedes aufgrund einer Pulswelle und für jedes aufgrund einer Verschiebungswelle in einem Blutgefäß verursachte Ultraschall-Dopplerfrequenzsignal erzeugt; und die Signalverarbeitungs-Schaltungsanordnung, welche der Ultraschall-Sende- und -Empfangseinrichtung zum Feststellen einer Gefäßwandbewegung nachgeschaltet ist, kann so ausgebildet sein, daß sie eine zweite Impulserzeugungseinrichtung, insbesondere eine Rechteckimpulserzeugungseinrichtung, ist, die für jedes Ultraschall-Dopplerfrequenzsignal, das durch eine Gefäßwandbewegung verursacht worden ist, welche ihrerseits durch eine Pulswelle in einem Blutgefäß hervorgerufen worden ist, je einen Impuls erzeugt. Diese Impulse, die vorzugsweise Rechteckimpulse sind, werden der Vergleichsstufe zugeführt, die im einfachsten Fall ein UND-Tor ist, das an seinem Ausgang nur dann ein Signal abgibt, wenn am Ausgang der ersten und zweiten Impulserzeugungseinrichtung gleichzeitig ein Impuls auftritt, so daß also das Ausgangssignal der Vergleichsstufe jeweils ein Systolen-Zählimpuls ist, der gegebenenfalls noch differenziert werden kann.

Außerdem können unter Umgehung der Vergleichsstufe die Impulse, die am Ausgang der ersten Impulserzeugungseinrichtung erscheinen, gegebenenfalls, nachdem sie differenziert worden sind, als Systolen- und Extrasystolen-Zählimpulse verwendet werden, und durch eine Differenzbildung zwischen den Systolen-Zählimpulsen und den Systolen- und Extrasystolen-Zählimpulsen kann man die Anzahl der ventrikulären Extrasystolen erhalten.

Vorteilhafterweise können die beiden Ultraschall-Sende- und -Empfangseinrichtungen in einem Meßkopf zusammengefaßt sein, wobei die Ultraschallsender in der Mitte einer parallel zu einer Ankopplungsmembran für die Ankopplung an die Körperoberfläche des Benutzers verlaufenden Querschnittsfläche des Meßkopfes angeordnet sind, während die Ultraschaltempfänger je in mehrere, vorzugsweise mindestens drei, miteinander parallelgeschaltete einzelne, gegenüber den Ultraschallsendern relativ kleinflächige Ultraschallempfänger aufgeteilt sind, die vorzugsweise symmetrisch um die Ultraschallsender herum angeordnet sind. Hierbei können die Empfänger, beispielsweise Piezokristalle, die zur Erfassung der Gefäßwandbewegung dienen, fokussiert und auf ihrer Rückseite im Unterschied zu den starr gelagerten Empfängern, beispielsweise Piezokristallen, für die Erfassung der Blutströmungsgeschwindigkeit, weich gelagert sein.

Sowohl aus der US-A-3 780 725 als auch aus der DE-A-1 905 620 sind Meßköpfe bekannt, in denen zwei Ultraschall-Sende- und -Empfangseinrichtungen in einem Meßkopf zusammengefaßt sind, wobei entweder die Sender und Empfänger jeweils gesonderte Schwinger sein können, deren Abstrahl- bzw. Empfangsrichtungen in spitzem Winkel zueinander verlaufen, wie das bei der Einrichtung nach der US-A-3 780 725 der Fall ist, oder es kann je ein sowohl als Sender als auch als Empfänger arbeitender Schwinger vorgesehen sein, wie in der DE-A-1 905 620 beschrieben.

Andere Weiterbildungen der Erfindung sind in Unteransprüchen angegeben und in der nachfolgenden Figurenbeschreibung erläutert.

Die Erfindung wird nachstehend unter Bezugnahme auf die Fig. 1 bis 6 der Zeichnung anhand eines besonders bevorzugten Ausführungsbeispiels näher erläutert; es zeigt

Fig. 1 ein Blockschaltbild einer Ausführungsform eines Kardiotachometers nach der Erfindung, wobei jedoch die Darstellung der Stromversorgung der einzelnen Einheiten aus Gründen der klareren Veranschaulichung der Funktionsweise dieses Kardiotachometers weggelassen worden ist,

Fig. 2 eine schematische Darstellung der Stromversorgung der einzelnen Einheiten des Kardiotachometers nach Fig. 1, wobei aus Vereinfachungsgründen nur einige Einheiten dieses Kardiotachometers dargestellt sind, während die Stromversorgung der übrigen Einheiten lediglich durch Pfeile angedeutet ist,

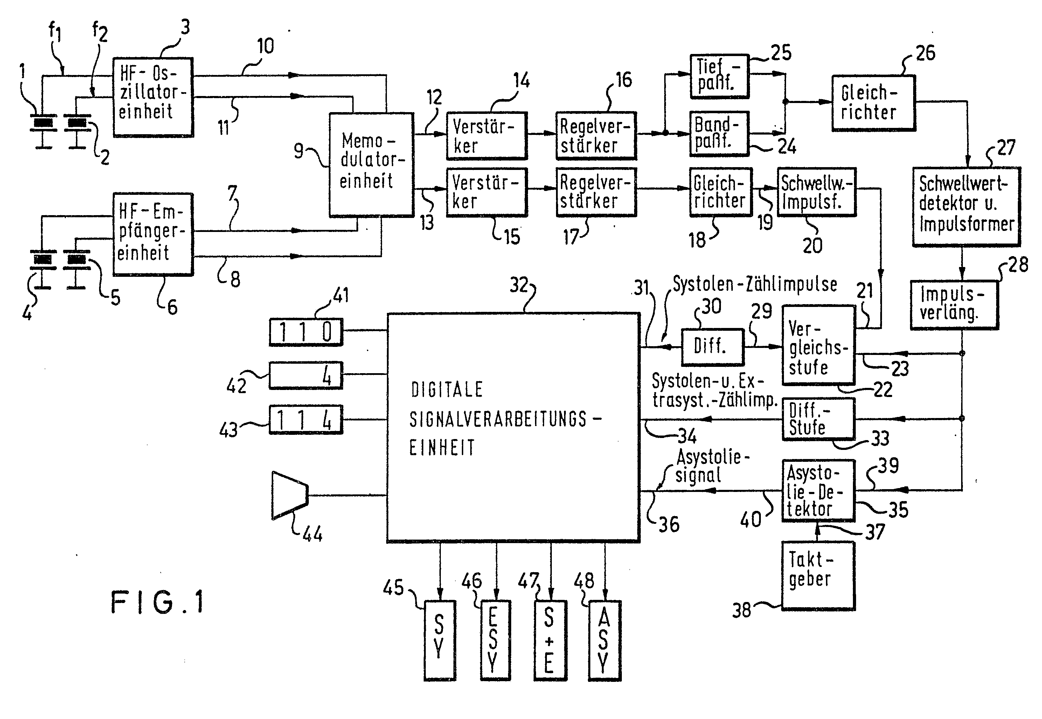

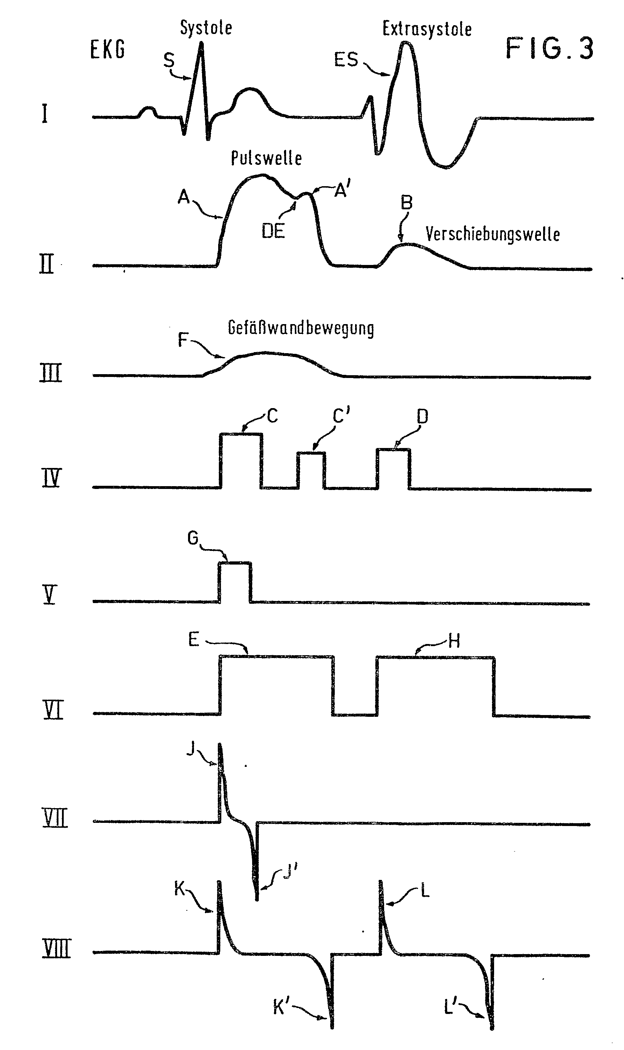

Fig. 3 eine Veranschaulichung eines Elektrokardiogramms mit einer Systole und einer willkürlich angenommenen Extrasystole sowie eine Pulswelle und eine Verschiebungswelle, die aufgrund der Systole bzw. der Extrasystole des Herzens in einer Arterie auftreten, und die Gefäßwandbewegung der Arterie, in der die Pulswelle auftritt, sowie weiterhin einzelne Signale, die aufgrund der Pulswelle, der Verschiebungswelle und der Gefäßwandbewegung in dem erfindungsgemäßen Kardiotachometer der Fig. 1 erzeugt werden, wobei die waagerechte Achse den zeitlichen Verlauf darstellt und dieser zeitliche Verlauf in den einzelnen Teilen I bis VIII der Fig. 3 übereinstimmt,

Fig. 4 eine schematische Aufsicht auf einen Meßkopf, der zwei Ultraschallsender und mehrere Ultraschallempfänger aufweist,

Fig. 5 einen Schnitt durch den Meßkopf der Fig. 4, in dem die Abstrahl- und Empfangswinkel der Ultraschallsender und -empfänger angedeutet sind, und

Fig. 6 einen Schnitt durch einen ähnlichen Meßkopf mit darunter im Abstand davon gezeichneter Hautoberfläche.

Es sei nun zunächst auf Fig. 1 Bezug genommen, wonach das dort dargestellte Ausführungsbeispiel eines Kardiotachometers einen ersten Ultraschallsender 1 und einen zweiten Ultraschallsender 2 aufweist, die beispielsweise Bariumtitanat-Schwinger sein können. Die Ultraschallsender 1 und 2 sind an eine Hochfrequenz-Oszillatoreinheit 3 angekoppelt. Diese Hochfrequenz-Oszillatoreinheit 3 erzeugt kontinuierliche Hochfrequenzschwingungen von zwei verschiedenen Frequenzen, und zwar von einer ersten Frequenz $f_1$, die im Bereich von 5 bis 10 MHz liegt und die vorzugsweise 5 MHz beträgt; und von einer zweiten Frequenz $f_2$, die im Bereich von 1 bis 3 MHz liegt und vorzugsweise 1 MHz beträgt.

Die Hochfrequenzschwingungen der Frequenz $f_1$ werden dem ersten Ultraschallsender 1 zugeführt, und der von diesem Ultraschallsender als kontinuierlicher Wellenzug abgestrahlte Ultraschall der gleichen Frequenz dient dazu, die Blutströmungsgeschwindigkeit durch Ultraschall-Dopplerfrequenz-Messung zu ermitteln. Dazu wird der Ultraschall vom Ultraschallsender 1 beispielsweise auf eine Arterie gerichtet, in der das Blut entsprechend den Herzschlägen der Person, bei der die Blutströmungsgeschwindigkeit gemessen wird, pulsiert. Die Blutströmungsgeschwindigkeit soll hierbei nicht absolut gemessen werden, sondern es soll vielmehr die Herzfrequenz erfaßt werden, d. h., es sollen Zählimpulse erzeugt werden, von denen jeder einer Systole des Herzens entspricht.

Hochfrequenzschwingungen der Frequenz $f_2$ werden dem zweiten Ultraschallsender 2 zugeführt, von dem sie in kontinuierliche Ultraschall-Wellenzüge der gleichen Frequenz umgewandelt werden. Diese Ultraschall-Wellenzüge dienen dazu, die Deformation eines Blutgefäßes bzw. des Gewebes festzustellen, die durch die Systolen des Herzens hervorgerufen werden, also zur Wandbewegungsdetektion durch Ultraschall-Dopplerfrequenz-Messung. Zu diesem Zweck ist der Ultraschallsender 2 beispielsweise auf die Arterie gerichtet, in der die Blutströmungsgeschwindigkeit gemessen wird, und zwar vorzugsweise auf die gleiche Stelle der Arterie, an der die weiter oben erwähnte Blutströmungsgeschwindigkeitsmessung erfolgt.

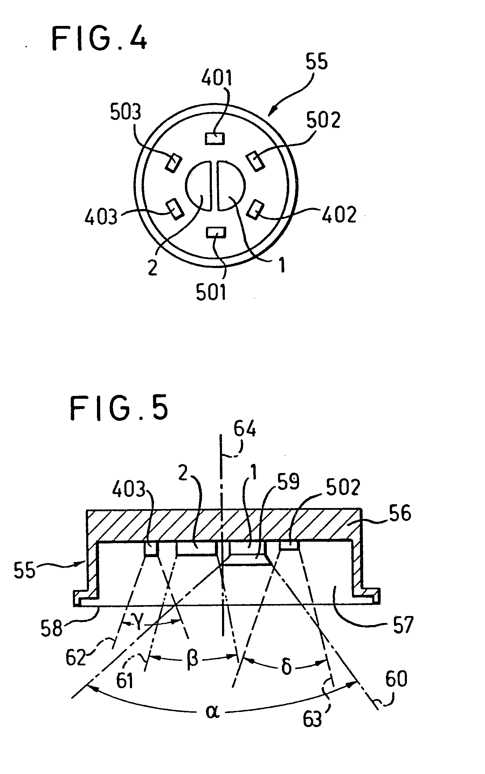

Weiterhin umfaßt das Kardiotachometer nach Fig. 1 einen ersten Ultraschallempfänger 4 und einen zweiten Ultraschallempfänger 5, die jeweils aus mehreren, miteinander verbundenen, einzelnen Ultraschallempfängern 501, 502 und 503 bzw. 401, 402 und 403 (siehe Fig. 4 und 5) bestehen können und die zusammen mit dem ersten und zweiten Ultraschallsender 1 bzw. 2 in einem gemeinsamen Meßkopf angeordnet sind, wie weiter unten näher erläutert wird. Diese Ultraschallempfänger 4 und 5, welche die empfangenen Ultraschallschwingungen in elektrische Hochfrequenzsignale umwandeln, können beispielsweise Bariumtitanat-Schwinger sein.

Die beiden Ultraschallempfänger 4 und 5 sind an eine Hochfrequenz-Empfängereinheit 6 angekoppelt, die so ausgebildet und abgestimmt ist, daß sie auf einer ersten Ausgangsleitung 7 die Hochfrequenzschwingungen der Frequenz $f_1$ liefert, die mit einer ersten Dopplerfrequenz $\Delta f_1$ moduliert sind, welche ihrerseits eine Funktion der Strömungsgeschwindigkeit des Blutes ist, und zwar ist diese Dopplerfrequenz $\Delta f_1$ der Strömungsgeschwindigkeit des Blutes weitgehend proportional. Außerdem liefert die Hochfrequenz-Empfängereinheit 6 auf einer zweiten Ausgangsleitung 8 ein elektrisches Hochfrequenzsignal von der Frequenz $f_2$, das mit einer zweiten Dopplerfrequenz $\Delta f_2$ moduliert ist, die aufgrund der Gefäßwandbewegung, beispielsweise der erwähnten Arterie, entsteht, und zwar ist die Dopplerfrequenz $\Delta f_2$ allgemein eine monotone Funktion der Gefäßwandbewegung, wobei wesentlich ist, daß durch diese Dopplerfrequenz $\Delta f_2$ nur qualitativ eine echte Gefäßdeformation bzw. -wandbewegung festgestellt werden soll, deren Größe oberhalb einer gewissen Schwelle liegt.

Die Ausgangsleitungen 7 und 8 der Hochfrequenz-Empfängereinheit 6 führen zu einer Demodulatoreinheit 9, der über Ausgangsleitungen 10 und 11 von der Hochfrequenz-Oszillatoreinheit 3 außerdem Hochfrequenzschwingungen der Frequenzen $f_1$ und $f_2$ zugeführt werden. In der Demodulatoreinheit 9 wird die Hochfrequenz-Trägerwelle der Frequenz $f_1$ bzw. $f_2$ weggenommen, so daß an einem ersten Ausgang 12 der Demodulatoreinheit 9 das Dopplerfrequenzsignal $\Delta f_1$ erscheint, während an einem zweiten Ausgang 13 dieser Demodulatoreinheit das Dopplersignal $\Delta f_2$ abgenommen werden kann. Es sei an dieser Stelle darauf hingewiesen, daß an sich sowohl positive als auch negative $\Delta f_1$- und $\Delta f_2$-Signale zur Auswertung bzw. zur Weiterverarbeitung in der Schaltungsanordnung nach Fig. 1 herangezogen werden können, obwohl im vorliegenden Fall bevorzugt positive $\Delta f_1$- und $\Delta f_2$-Signale ausgewertet werden.

Das $\Delta f_1$-Signal wird über einen ersten Verstärker 14 verstärkt, und das $\Delta f_2$-Signal wird über einen zweiten Verstärker 15 verstärkt. Jedem dieser beiden Verstärker 14 und 15 ist jeweils ein automatischer Regelverstärker 16 bzw. 17 mit Spitzenwertdetektor nachgeschaltet, so daß man an den Ausgängen dieser Regelverstärker 16, 17 Wellenzüge erhält, deren Signalspannungshöhe durch die Regelverstärker jeweils auf etwa gleiches Niveau gebracht ist und deren Frequenz jeweils gleich der Dopplerfrequenz $\Delta f_1$ bzw. $\Delta f_2$ ist; an dieser Stelle sei darauf hingewiesen, daß die Dopplerfrequenzen $\Delta f_1$ und $\Delta f_2$ natürlich keine genau diskreten Frequenzen sind, sondern je ein ganzes Frequenzspektrum umfassen, weil die Blutströmungsgeschwindigkeit und die Geschwindigkeit der Gefäßwandbewegung ebenfalls aus einem ganzen Spektrum von Geschwindigkeiten bestehen, so daß notwendigerweise jeweils auch ein ganzes Spektrum von Dopplerfrequenzen erzeugt wird.

Dem Regelverstärker 17 ist ein Gleichrichter 18 nachgeschaltet, an dessen Ausgang 19 das bei III in Fig. 3 dargestellte Signal F erhalten wird, welches die Gefäßwandbewegung repräsentiert. Dieses Signal F wird durch eine dem Gleichrichter 18 nachgeschaltete Schwellwertdetektor- und Impulsformereinheit 20 in einen Rechteckimpuls G umgewandelt, der in Fig. 3 bei V dargestellt ist. Der Ausgang der Schwellwertdetektor- und Impulsformereinheit 20 ist mit einem ersten Eingang 21 einer Vergleichsstufe 22 verbunden, auf die weiter unten näher eingegangen wird, nachdem zunächst nachstehend näher erörtert wird, in welcher Weise die Signale, die am Ausgang des Regelverstärkers 16 auftreten, zu Eingangssignalen für den zweiten Eingang 23 der Vergleichsstufe 22 verarbeitet werden.

Zum Verständnis der auf den Regelverstärker 16 folgenden weiteren Signalverarbeitung ist es zunächst erforderlich, auf die Teile I und II der Fig. 3 näher einzugehen. Bei I ist in Fig. 3 ein Teil eines Elektrokardiogramms dargestellt, das von irgendeinem Elektrokardiographen aufgenommen worden ist und einen Kurvenverlauf S enthält, der einer Systole eines menschlichen Herzens entspricht, sowie einen nachfolgenden Kurvenverlauf ES, der einer willkürlich angenommenen Extrasystole entspricht, welche in einem gewissen zeitlichen Abstand auf die Systole folgen möge. Während nun der Systole des Herzens eine Pulswelle in einer Arterie entspricht, kommt es bei einer Extrasystole nur zu einer sogenannten Verschiebungswelle in der Arterie. Das Spektrum der Dopplerfrequenzen einer Pulswelle unterscheidet sich nun wesentlich von dem Spektrum der Dopplerfrequenzen einer Verschiebungswelle, und zwar liegen die Frequenzen des ersteren Spektrums im Bereich zwischen 700 und 1800 Hz, während die Frequenzen des letzteren Spektrums im Bereich von etwa 0 bis 500 Hz liegen. Um unerwünschte Störgeräusche, die auch als Artefakten bezeichnet werden und beispielsweise durch Kratzen verursacht werden oder Rundfunkgeräusche sein können, auszuschalten, ist dem Regelverstärker 16 zum Herausfiltern des Frequenzspektrums, welches den durch eine Pulswelle verursachten Dopplerfrequenzen entspricht, ein Bandpaßfilter 24 nachgeschaltet, das nur Frequenzen zwischen 700 und 1800 Hz durchläßt.

Außerdem ist dem Regelverstärker 16 ein Tiefpaßfilter 25 nachgeschaltet, das parallel zu dem Bandpaßfilter 25 liegt und die Frequenzen durchläßt, die in dem Frequenzspektrum der einer Verschiebungswelle entsprechenden Dopplerfrequenzen liegen. Dieses Tiefpaßfilter hat also einen Durchlaßbereich, der zwischen 0 und 500 Hz liegt. Zwar können in diesem Bereich auch eine Reihe von Störgeräuschen auftreten, aber die Tatsache, ob es sich im einzelnen Fall um ein Störgeräusch oder ein echtes, durch eine Verschiebungswelle verursachtes Signal handelt, läßt sich dadurch feststellen, ob unmittelbar vorher eine Pulswelle aufgetreten ist oder nicht. Eine entsprechende Schaltung ist, um die vorliegende Ausführungsform nicht unnötig zu komplizieren, in der Zeichnung nicht dargestellt, zumal eine solche zusätzliche Schaltung in vielen Fällen wegen geringen Störgeräuschpegels wegfallen kann.

Dem Bandpaßfilter 24 und dem Tiefpaßfilter 25 ist ein gemeinsamer Gleichrichter 26 nachgeschaltet, an dessen Ausgang die bei II in Fig. 3 dargestellten Signale A und B auftreten, von denen ersteres einer Pulswelle und letzteres einer Verschiebungswelle entspricht. Das insgesamt mit A bezeichnete Signal kann noch einen Signalteil A′ haben, auf den weiter unten näher eingegangen wird. Da die Signale A und B stets nacheinander und nie gleichzeitig auftreten können, braucht dem Bandpaßfilter 24 und dem Tiefpaßfilter 25 nicht je ein gesonderter Gleichrichter nachgeschaltet zu werden, sondern es genügt, wie erwähnt, der gemeinsame Gleichrichter 26.

Auf den Gleichrichter 26 folgt eine Schwellwertdetektor- und Impulsformereinheit 27, welche die Signale A und B in Rechteckimpulse umwandelt, so daß am Ausgang dieser Schwellwertdetektor- und Impulsformereinheit die bei IV in Fig. 3 dargestellten Rechteckimpulse C und D erscheinen sowie gegebenenfalls außerdem der Rechteckimpuls C′, der dem Kurvenverlauf A′ entspricht. Der Signalteil A′, der zu einem zusätzlichen Rechteckimpuls C′ führt, wird dadurch verursacht, daß die Pulswelle normalerweise ein Zwischenminimum DE aufweist, das als Dicrotie bezeichnet wird und vom Klappenschluß der Aorten-Herzklappe herrührt. Diese Dicrotie kann auch praktisch fehlen, beispielsweise dann, wenn Klappeninsuffizienzen vorliegen. Der auf die Dicrotie DE folgende Anstieg, welchem der Signalteil A′ entspricht, kann größer oder kleiner sein, was davon abhängt, wie gut die Elastizität des jeweiligen Herzens und des peripheren Gefäßsystems ist. Beim normalen gesunden Herzen eines Menschen tritt jedenfalls im allgemeinen eine merkliche Dicrotie auf, so daß in diesen Fällen jeweils ein zusätzlicher Rechteckimpuls C′ am Ausgang der Schwellwertdetektor- und Impulsformereinheit 27 entsteht.

Dieser zusätzliche Rechteckimpuls C′ ist aber unerwünscht, weil man für jede Pulswelle, die einer Systole entspricht, nur einen einzigen Rechteckimpuls erhalten will, um die Systolen unabhängig von dem jeweiligen individuellen Benutzer des Kardiotachometers eindeutig zählen zu können. Aus diesem Grunde ist der Schwellwertdetektor- und Impulsformereinheit 27 ein Impulsverlängerer 28 nachgeschaltet, der den Rechteckimpuls C bis auf eine Breite von 300 msec verlängert, so daß man den bei VI in Fig. 3 dargestellten Rechteckimpuls E erhält, der gewissermaßen den zusätzlichen Rechteckimpuls C′ »verschluckt« hat. Die Impulsbreite von 300 msec ergibt sich aufgrund der Tatsache, daß die maximale zeitliche Breite der Pulswelle den Wert von 300 msec nicht überschreitet, so daß bei allen möglicherweise auftretenden Pulswellen ein gegebenenfalls vorhandener zusätzlicher Rechteckimpuls C′ von dem Rechteckimpuls E »verschluckt« wird. Dieser Rechteckimpuls E und der zwangsläufigerweise auch auf 300 msec zu dem Rechteckimpuls H verbreiterte, einer Verschiebungswelle entsprechende Rechteckimpuls D werden auf den zweiten Eingang 23 der Vergleichsstufe 22 gegeben.

Die Vergleichsstufe 22 kann zum Beispiel ein UND-Tor sein, das die beiden Eingänge 21 und 23 hat, von denen der eine das bei V in Fig. 3 dargestellte Signal und der andere die bei VI in Fig. 3 angedeuteten Signale erhält. Am Ausgang 29 dieses UND-Tores bzw. der Vergleichsstufe 22 erhält man infolgedessen nur dann ein Signal in Form eines Rechteckimpulses, wenn gleichzeitig die Signale G und E auftreten, d. h., wenn eine echte Pulswelle auftritt, die dadurch charakterisiert ist, daß gleichzeitig mit ihr eine merkliche Gefäßwandbewegung stattfindet. Wenn dagegen das Signal H auftritt, das einer Verschiebungswelle entspricht, die dadurch charakterisiert ist, daß gleichzeitig mit ihr keine merkliche Gefäßwandbewegung stattfindet, dann erscheint am Ausgang 29 kein Signal. Da eine Pulswelle einer Systole und eine Verschiebungswelle einer Extrasystole entsprechen, wird am Ausgang 29 nur für jede Systole ein Rechteckimpuls erhalten, der in einer Differenzierstufe 30 differenziert wird, so daß man an einem ersten Eingang 31 einer digitalen Signalverarbeitungseinheit 32, an welchen der Ausgang der Differenzierstufe 30 angeschlossen ist, für jede Systole einen positiven und negativen Nadelimpuls I und I′ erhält, von denen aber nur ersterer als Zählimpuls wirksam wird, weil die auf den Eingang 31 in der digitalen Signalverarbeitungseinheit 32 angeschlossene Elektronik in entsprechender Weise flankengetriggert ist. Effektiv erhält man also am Eingang 31 Systolen-Zählimpulse.

Der Ausgang des Impulsverlängerers 28 ist außerdem an den Eingang einer weiteren Differenzierstufe 33 angeschlossen, deren Ausgang mit einem zweiten Eingang 34 der digitalen Signalverarbeitungseinheit 32 verbunden ist. Die Differenzierstufe 33 differenziert die Rechteckimpulse E und H, so daß man am Eingang 34 für jeden Rechteckimpuls E und H einen positiven und negativen Nadelimpuls K bzw. K′ und L bzw. L′ erhält, von denen wegen entsprechender Flankentriggerung der auf den Eingang 34 folgenden Elektronik der digitalen Signalverarbeitungseinheit 32 nur die positiven Nadelimpulse K und L wirksam werden; letztere bilden also effektiv Systolen- und Extrasystolen-Zählimpulse, so daß die Summe der am Eingang 34 wirksam werdenden Impulse gleich der Summe der bei dem Träger des Kardiotachometers stattfindenden Systolen und Extrasystolen ist.

Schließlich ist der Ausgang des Impulsverlängerers 28 außerdem noch mit dem Eingang eines sogenannten Asystolie-Detektors 35 verbunden. Dieser Asystolie-Detektor 35 gibt an seinem Ausgang, der mit einem dritten Eingang 36 der digitalen Signalverarbeitungseinheit 32 verbunden ist, immer dann ein Signal ab, wenn während einer vorbestimmten Zeitdauer, die vorzugsweise zu 1200 msec gewählt wird, weder eine Systole noch eine Extrasystole auftritt, also während 1200 msec weder ein Rechteckimpuls E noch ein Rechteckimpuls H am Eingang des Asystolie-Detektors 35 erscheint. Zu diesem Zweck kann der Asystolie-Detektor 35 beispielsweise als Zähler ausgebildet sei, der an einem Zähleingang 37 von einem Taktgeber 38 laufend Zählimpulse erhält und dessen an den Impulsverlängerer 28 angeschlossener Eingang 39 der Rücksetzeingang des Zählers ist, während der an den Eingang 36 der digitalen Signalverarbeitungseinheit 32 angeschlossene Ausgang 40 des Asystolie-Detektors dann der Zählausgang dieses Zählers ist, an dem nur dann ein Signal erscheint, wenn ein vorbestimmter Zählwert erreicht wird, der einem Nichtauftreten von Systolen und Extrasystolen während einer vorbestimmten Zeitdauer von beispielsweise 1200 msec entspricht. Dieser Zählwert, bei dem ein Signal am Ausgang 40 erscheint, beträgt im angegebenen Beispielsfall 1200, sofern der Taktgeber 38 pro Millisekunde einen Zählimpuls an den Zähleingang 37 abgibt, denn der Zählwert 1200 wird in diesem Fall nur dann erreicht, wenn der Zähler, welcher den Asystolie-Detektor 35 bildet, nicht innerhalb von 1200 msec durch einen Rechteckimpuls E oder H zurückgestellt wird. Das am Ausgang 40 auftretende Signal wird nachstehend auch als Asystoliesignal bezeichnet.

Die digitale Signalverarbeitungseinheit 32 braucht im einzelnen nicht näher beschrieben zu werden, da sie im Prinzip ein Rechner mit Zeitmeßeinrichtung sein kann und sich ihr Aufbau aus den nachfolgend dargelegten Funktionen für den Fachmann ohne weiteres ergibt.

In dem dargestellten Ausführungsbeispiel hat die digitale Signalverarbeitungseinheit 32 die Funktionen, aufgrund der an den Eingängen 31, 34 und 36 auftretenden Signale folgendes zu tun:

(1) Die Herzpulsfrequenz zu ermitteln und sie auf eine erste Anzeigeeinrichtung 41 zu geben, auf der die Herzpulsfrequenz in Schlägen pro Minute angezeigt wird. Im vorliegenden Beispiel zeigt die Anzeigeeinrichtung 41 gerade 110 Herzschläge pro Minute an. Diese Herzpulsfrequenz kann dadurch ermittelt werden, daß in der digitalen Signalverarbeitungseinheit aufgrund der Systolen-Zählimpulse, die am Eingang 31 erhalten werden, die Periodendauer zwischen zwei Systolen gemessen wird. Die Herzpulsfrequenz beträgt im allgemeinen zwischen etwa 40 und etwa 180 Schlägen pro Minute.

(2) Weiterhin ermittelt die digitale Signalverarbeitungseinheit 32 die Anzahl von Extrasystolen, die während einer vorbestimmten Zeitdauer, beispielsweise während einer Minute, im Mittel auftreten. Diese mittlere Anzahl von Extrasystolen pro Minute wird auf einer zweiten Anzeigeeinrichtung 42 digital angezeigt. Um die medizinische Wichtigkeit dieser Größe deutlich zu machen, sei darauf hingewiesen, daß dann, wenn nur ganz wenige Extrasystolen auftreten, beispielsweise im Mittel eine einzige Extrasystole pro Stunde, noch keine Gefährdung für die Person vorliegt, die das Kardiotachometer benutzt; wenn dagegen aber beispielsweise im Mittel in einer Minute bereits sieben Extrasystolen auftreten, dann ist eine Gefährdung für diese Person gegeben. Nach der beispielsweisen Darstellung der Fig. 1 zeigt die Anzeigeeinrichtung 42 gerade an, daß im Mittel vier Extrasystolen pro Minute auftreten. Diese Anzahl von Extrasystolen läßt sich, wie ohne weiteres ersichtlich ist, durch Differenzbildung der am Eingang 31 und am Eingang 34 erhaltenen Zählimpulse in Verbindung mit einer Zeitmessung gewinnen.

(3) Außerdem soll die digitale Signalverarbeitungseinheit 32 an einer dritten Anzeigeeinrichtung 43 die Anzahl der im Mittel pro Zeiteinheit auftretenden Systolen und Extrasystolen zur Anzeige bringen. Dieser Anzeigewert ist, wenn sich die Anzeigewerte, die auf den Anzeigeeinrichtungen 41 und 42 erscheinen, auf die gleiche Zeiteinheit beziehen, gleich der Summe der letzteren beiden Anzeigewerte. Im vorliegenden Beispielsfall ist also im Mittel die Summe von Systolen und Extrasystolen pro Minute gleich 114.

(4) Es soll ein Alarmsignal erzeugt werden, wenn die Herzpulsfrequenz außerhalb eines einstellbaren Bereiches liegt, der individuell verschieden ist. Dieses Alarmsignal kann als akustisches Signal mit einem Lautsprecher 44 erzeugt werden. Da jedoch verschiedene Alarmsignale erzeugt werden sollen und der Benutzer des Kardiotachometers jeweils in der Lage sein soll, eindeutig festzustellen, welcher Alarmzustand gerade gegeben ist, ist außerdem eine erste optische Alarmanzeigeeinrichtung 45 vorgesehen, auf der beispielsweise die Anzeige »SY« erscheint, wenn die Herzpulsfrequenz unter- oder oberhalb des vorerwähnten einstellbaren Bereiches liegt. Dieser einstellbare Bereich beträgt bei einem gesunden Menschen zum Beispiel 40 bis 180 Herzschläge pro Minute, so daß bei diesem Menschen ein Alarmsignal erst ausgelöst wird, wenn die Herzpulsfrequenz unter 40 oder über 180 liegt. Dagegen kann bei einem Infarktgeschädigten der zulässige Herzpulsfrequenzbereich so liegen, daß es erforderlich ist, ein Alarmsignal schon dann auszulösen, wenn die Herzpulsfrequenz unter 70 oder über 120 liegt.

(5) Weiterhin soll die digitale Signalverarbeitungseinheit 32 ein Alarmsignal auslösen, wenn die mittlere Anzahl der Extrasystolen einen vorbestimmten Wert, der beispielsweise sechs Extrasystolen pro Minute beträgt, überschreitet. Dieses Alarmsignal wird ebenfalls akustisch durch den Lautsprecher 44 erzeugt, und es wird gleichzeitig an einer zweiten optischen Alarmanzeigeeinrichtung 46 angezeigt, indem auf dieser beispielsweise die Anzeige »ESY« erscheint.

(6) Schließlich soll ein Alarmsignal ausgelöst werden, wenn die im Mittel pro Zeiteinheit auftretende Summe von Systolen und Extrasystolen einen vorbestimmten Wert überschreitet. Dieses Alarmsignal wird außer durch den Lautsprecher 44 noch durch eine dritte optische Alarmanzeigeeinrichtung 47 gegeben, auf der in einem solchen Fall zum Beispiel die Anzeige »S + E« erscheint. Diese mittlere Anzahl von Systolen plus Extrasystolen kann beispielsweise diejenige Anzahl sein, die im Mittel während fünf Normalschlägen des Herzens auftritt.

(7) Endlich soll ein Alarmsignal abgegeben werden, wenn während einer vorbestimmten Zeitdauer von beispielsweise 1200 msec jede Systole und Extrasystole überhaupt ausbleibt. Dieses Signal ist praktisch das am Eingang 36 auftretende Asystoliesignal und wird akustisch durch den Lautsprecher 44 und optisch durch eine vierte Alarmanzeigeeinrichtung 48 abgegeben, wobei zum Beispiel im Alarmfall auf letzterer die Anzeige »ASY« erscheint.

Obwohl aus Gründen der klareren Darstellung drei verschiedene Ziffernanzeigeeinrichtungen 41, 42 und 43 in Fig. 1 gezeigt sind, kann stattdessen auch eine einzige Ziffernanzeigeeinrichtung verwendet werden, auf der wahlweise die einzelnen Anzeigewerte erscheinen, beispielsweise auf Knopfdruck hin oder automatisch in periodischer Aufeinanderfolge.

Auch die optischen Alarmanzeigeeinrichtungen 45, 46, 47 und 48 können, was schon aus Raum- und Kostengründen zu bevorzugen ist, durch eine einzige optische Alarmanzeigeeinrichtung ersetzt werden, auf der dann jeweils im Alarmfall die Anzeige »SY« oder »ESY« oder »S + E« oder schließlich »ASY« erscheint.

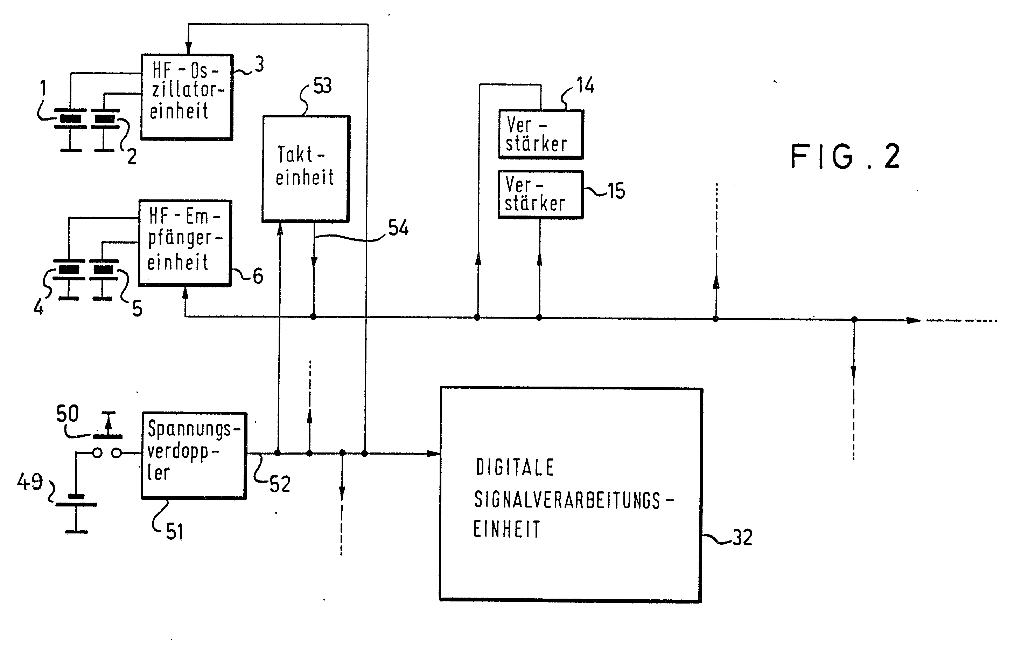

In Fig. 2 ist die Stromversorgung des in Fig. 1 dargestellten Kardiotachometers schematisch dargestellt. Diese umfaßt, da das Kardiotachometer vorzugsweise in Form einer Armbanduhr ausgebildet ist, eine kleine Batterie 49, beispielsweise eine Lithiumbatterie. Diese Batterie 49 ist über einen Schalter 50, beispielsweise einen Druckschalter, an einen Spannungsverdoppler 51 angeschlossen, der deswegen vorgesehen ist, weil die geringe Batteriespannung, beispielsweise die Spannung der Lithiumbatterie von nur 2,6 Volt, nicht ausreicht, um die einzelnen Schaltungseinheiten des Kardiotachometers zu betreiben. Der Ausgang 52 des Spannungsverdopplers 51 ist einerseits mit allen den Schaltungseinheiten der Fig. 1 verbunden, die dauernd an ihre Betriebsspannung angeschlossen sein sollten, wie zum Beispiel die Hochfrequenz-Oszillatoreinheit 3 und die digitale Signalverarbeitungseinheit 32. Andererseits ist der Ausgang 52 des Spannungsverdopplers 51 an den Eingang einer Takteinheit 53 angeschlossen, an deren Ausgang 54 die Versorgungsspannung für andere Schaltungseinheiten des Kardiotachometers nach Fig. 1 nur im Takt der Herzfrequenz während der Zeitperioden eingeschaltet ist, während der Systolen und Extrasystolen auftreten können. Dagegen ist die Versorgungsspannung am Ausgang 54 in der übrigen Zeit ausgeschaltet, um der Batterie 49 eine längere nutzbare Lebensdauer zu geben. Um den dadurch erzielbaren Vorteil deutlich zu machen, sei darauf hingewiesen, daß die Zeit, die zwischen dem Ende einer Pulswelle und dem Beginn der darauffolgenden Pulswelle verstreicht, im Mittel etwa 500 msec beträgt. Wenn man weiterhin davon ausgeht, daß Extrasystölen kurz nach einer normalen Systole auftreten, läßt sich durch die Takteinheit 53 eine erhebliche Verminderung des Stromverbrauches des Kardiotachometers erreichen.

An den Ausgang 54 der Takteinheit 53 sind beispielsweise die Hochfrequenz-Empfängereinheit 6, die Demodulatoreinheit 9, die beiden Verstärker 14 und 15, das Bandpaßfilter 24, der Gleichrichter 26, die Schwellwertdetektor- und Impulsformereinheit 27 und die Differenzierstufe 30 angeschlossen. Darüber hinaus können auch noch weitere Schaltungseinheiten der Fig. 1 an den Ausgang 54 angeschlossen sein.

Im allgemeinen ist es möglich, die Stromversorgung, soweit sie über die Takteinheit 53 ein- und ausgeschaltet wird, für etwa ein Drittel der Meßzeit abzuschalten.

Die Schaltung des Kardiotachometers nach Fig. 1 kann im Rahmen der Erfindung auch auf verschiedene Weise abgewandelt werden. So ist es möglich, der digitalen Signalverarbeitungseinheit 32 anstatt über deren Eingang 34 Systolen und Extrasystolen-Zählimpulse zuzuführen, auf diesen Eingang nur Extrasystolen-Zählimpulse zu geben, indem die am Ausgang des Bandpaßfilters 24 und am Ausgang des Tiefpaßfilters 25 auftretenden Signale gesondert weiterbehandelt werden. Das kann etwa dadurch geschehen, daß nur die Signale, die am Ausgang des Bandpaßfilters 24 auftreten, über den Gleichrichter 26 und die Schwellwertdetektor- und Impulsformereinheit 27 sowie den Impulsverlängerer 28 weiterbehandelt werden und der Ausgang des Impulsverlängerers 28 zwar mit dem Eingang 23 der Vergleichsstufe 22 und dem Eingang 39 des Asystolie-Detektor 35, nicht jedoch mit dem Eingang der Differenzierstufe 33 verbunden ist; während dagegen die Signale, die am Ausgang des Tiefpaßfilters 25 erscheinen, über einen gesonderten Gleichrichter und eine diesem nachgeschaltete, gesonderte Schwellwertdetektor- und Impulsformereinheit, deren Ausgang mit dem Eingang der Differenzierstufe 33 vorhanden ist, weiterbehandelt werden. Diese Ausführungsform ist jedoch etwas aufwendiger als die in Fig. 1 gezeigte Ausführung des Kardiotachometers, weil eine Differenzbildung zwischen der Anzahl der Systolen-Zählimpulse, die am Eingang 31 erscheinen, und der Anzahl der Systolen- und Extrasystolen-Zählimpulse, die am Eingang 37 erscheinen, in der digitalen Signalverarbeitungseinheit zur Ermittlung der Anzahl der Extrasystolen weniger Schaltungsaufwand und Energieverbrauch bedeutet als ein zusätzlicher Gleichrichter und eine zusätzliche Schwellwertdetektor- und Impulsformereinheit.

Auch können der Asystoliedetektor 35 und der Taktgeber 38, die aus Gründen ihrer besonderen Wichtigkeit gesondert dargestellt worden sind, in die digitale Signalverarbeitungseinheit 32 einbezogen sein, wobei dann der Asystoliedetektor 35 jeweils durch die Systolen- und Extrasystolen-Zählimpulse zurückgestellt wird.

Es sei nun auf die Fig. 4 und 5 Bezug genommen, in denen ein bevorzugtes Ausführungsbeispiel eines Meßkopfes 55 dargestellt ist, der den ersten Ultraschallsender 1, den zweiten Ultraschallsender 2 sowie den ersten Ultraschallempfänger 4 und den zweiten Ultraschallempfänger 5 des Kardiotachometers umfaßt, wobei der erste Ultraschallempfänger 4 aus vorzugsweise mindestens drei parallelgeschalteten Ultraschallempfängern 401, 402 und 403 und der zweite Ultraschallempfänger 5 ebenfalls vorzugsweise aus mindestens drei parallelgeschalteten Ultraschallempfängern 501, 502 und 503 bestehen.

Die Ultraschallsender 1 und 2 sind relativ großflächige Wandlerelemente, vorzugsweise Piezokristalle, die beispielsweise etwa halbkreisförmig ausgebildet und in der Mitte der der Hautoberfläche des Trägers zugewandten Querschnittsfläche des Meßkopfes 55 angeordnet sind. Demgegenüber sind die Ultraschallempfänger 401, 402 und 403 sowie 501, 502 und 503 relativ kleinflächige Wandler, vorzugsweise Piezokristalle, die um die beiden Ultraschallsender 1 und 2 herum regelmäßig, und zwar vorzugsweise je für sich symmetrisch, angeordnet sind, so daß also die einzelnen Ultraschallempfänger in der dargestellten Ausführungsform in gleichen Abständen auf einem Kreisumfang verteilt sind, wobei die Ultraschallempfänger 401, 402 und 403 an den Spitzen eines ersten gleichseitigen Dreiecks liegen, während die Ultraschallempfänger 501, 502 und 503 an den Spitzen eines zweiten gleichseitigen Dreiecks angeordnet sind.

Die Ultraschallsender und -empfänger sind in einem Trägerkörper 56 befestigt, vorzugsweise einen aus Epoxyharz bestehenden Trägerkörper 56 eingegossen, da dieser eine gute Schwingungsisolation nach der Rückseite des Meßkopfes 55 hin gewährleistet und durch denselben eine gute Verkapselung der Ultraschallsender und -empfänger erreicht wird. Mit ihren Abstrahl- bzw. Aufnahmeseiten für den Ultraschall ragen die Ultraschallsender und -empfänger in ein Koppelmedium 57 hinein, das beispielsweise Öl oder ein Gel sein kann. Schließlich wird der Meßkopf 55 auf seiner dem Träger des Kardiotachometers zugewandten Seite durch eine Membran 58 abgeschlossen, die den Hautkontakt mit dem Träger herstellt, und zwar vorzugsweise über ein weiteres Koppelmedium, das zwischen der Haut des Trägers und der Membran 58 des Meßkopfes 55 vorgesehen ist.

Vor dem Sender 1, der zur Messung der Strömungsgeschwindigkeit des Blutes in einem oder mehreren Blutgefäßen dient, befindet sich eine vorzugsweise aus Kunststoff bestehende Linse 59, welche den Ultraschall-Abstrahlkegel 60 dieses Ultraschallsenders 1 auf einen Öffnungswinkel $\alpha$ von wesentlich mehr als 90°, vorzugsweise auf 120°, erweitert. Dadurch wird einerseits sichergestellt, daß ohne spezielle bzw. ohne allzu genaue Befestigung und Lokalisierung des Kardiotachometers mit größter Wahrscheinlichkeit ein entsprechendes Blutgefäß, beispielsweise die arteria radialis, und einige weitere größere arterielle Gefäße beschallt werden; da die zu messende Pulswelle in allen vom Ultraschall-Abstrahlkegel 60 getroffenen arteriellen Gefäßen synchron auftritt, macht es nichts aus, wenn mehrere arterielle Gefäße gleichzeitig erfaßt werden, sondern das ist im Gegenteil vorteilhaft. Die dabei zwangsweise beschallten venösen Gefäße stören in ihrer reflektierten Ultraschall-Energie insofern nicht, als die Strömungsgeschwindigkeiten in ihnen wesentlich kleiner sind und die dadurch erzeugten Dopplerfrequenzen somit außerhalb der Mittenfrequenz des Bandpaßfilters 24 liegen.

Der Ultraschallsender 2 zur Ermittlung der Wandbewegung des jeweiligen Blutgefäßes, in dem die Pulswelle auftritt, beispielsweise der arteria radialis, hat hingegen einen Ultraschall-Abstrahlkegel 61, dessen Öffnungswinkel $\beta$ wesentlich geringer als 90° ist, und zwar vorzugsweise etwa 60° beträgt. Ein solcher relativ enger Öffnungwinkel des Ultraschall-Abstrahlkegels 61 ist deswegen notwendig, weil bei Muskelkontraktionen des Unterarms und im Bereich des Handwurzelknochens (ullna, radius) keine Signale aufgenommen werden sollen, sondern vielmehr nur die von der Wandbewegung des interessierenden Gefäßes herkommenden Signale empfangen werden sollen.

Weiterhin ist der Ultraschallsender 1 für die Ermittlung der Strömungsgeschwindigkeit vorzugsweise so angebracht, daß sein Neigungswinkel retrograd verläuft, d. h., daß das skalare Produkt aus dem Schallausbreitungsvektor und dem Strömungsvektor kleiner als 0 ist, also zum Herzen hin gewandt ist und große Gefäße erfaßt werden.

Die Ultraschall-Empfangskegel 62 der Ultraschallempfänger 401, 402 und 403 sowie die Ultraschall-Empfangskegel 63 der Ultraschallempfänger 501, 502 und 503 sind vorzugsweise in ihrem natürlichen Öffnungswinkel $\gamma$ bzw. $\delta$ von etwa 90° nicht verändert, jedoch in ihrer Anordnung zu den Ultraschallsendern 1 und 2 unter einem Neigungswinkel von 15 bis 30°, vorzugsweise von 15°, zu der senkrecht zur Membran 58 verlaufenden Mittelachse 64 des Meßkopfes 55 befestigt. Damit wird erreicht, daß bei nicht genauer Anordnung des Meßkopfes über dem jeweils zu erfassenden Blutgefäß, beispielsweise der arteria radialis, mit Sicherheit für die Weiterverarbeitung geeignete Signale erfaßt werden. Durch unterschiedliche Wandlagerung und Resonanzfrequenz der Ultraschallempfänger 401, 402 und 403 gegenüber den Ultraschallempfängern 501, 502 und 503 wird ein Übersprechen (akustische Kopplung) und eine Störung durch harmonische Wellen weitgehend ausgeschlossen.

Die Anordnung der Ultraschallsender kann auch so sein, daß der Neigungswinkel, d. h. der Winkel zwischen Haupt-Schallausbreitungsrichtung und Strömungsrichtung bei den Ultraschallempfängern 401, 402 und 403 für die Strömungsdetektion 15 bis 30° beträgt, während der Neigungswinkel der Ultraschallempfänger 501 502 und 503 für die Wandbewegungsdetektion 30 bis 45° zur Körperoberfläche beträgt.

Der in den Fig. 4 und 5 dargestellte Meßkopf 55 kann insbesondere dahingehend abgewandelt werden, daß die Ultraschallempfänger 4 und 5 in mehr als drei einzelne, miteinander parallelgeschaltete Ultraschallempfänger aufgeteilt werden.

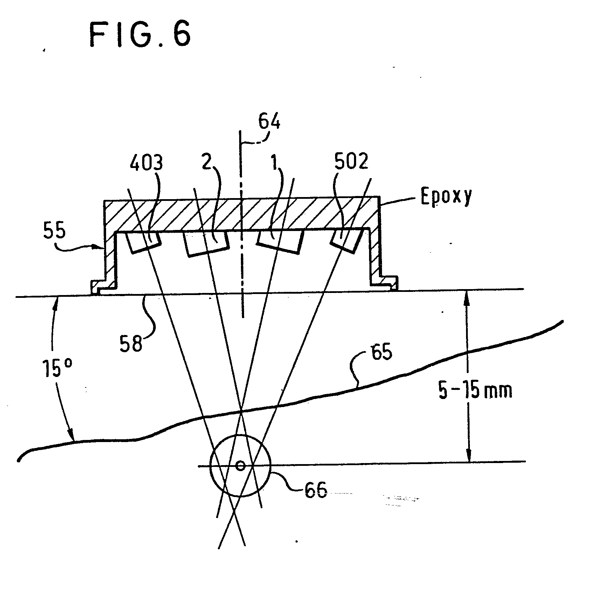

In Fig. 6 sind die Winkel besonders gut, unter denen die Ultraschallsender und -empfänger gegenüber der Hautoberfläche geneigt sind. Die Neigung der Abstrahl- bzw. Empfangsflächen gegenüber der Hautoberfläche ist gleich der Neigung der Hauptabstrahl- bzw. -empfangsrichtungen gegenüber der Haupt- bzw. Mittelachse 64, die auf der Membran 58 senkrecht steht. Diese Neigung liegt erfindungsgemäß zwischen etwa 15° und 30° und kann einstellbar gemacht sein.

Die Hautoberfläche ist in Fig. 6 mit 65 bezeichnet, wobei allerdings der Meßkopf 55 abgehoben von der Hautoberfläche 65 dargestellt ist. Mit 66 ist die arteria radialis bezeichnet. Man sieht, daß die Ultraschallsender und -empfänger auf diese Ader konzentriert gerichtet sind.

**Patentansprüche**

1. Kardiotachometer zum Ermitteln der Herzfrequenz unter Berücksichtigung von ventrikulären Extrasystolen mittels einer nach dem Ultraschall-Doppler-Prinzip arbeitenden Ultraschall-Sende- und -Empfangseinrichtung (2, 5) mit nachgeschalteter Signalverarbeitungs-Schaltungsanordnung (9, 15, 17, 18, 20) zum Feststellen einer Gefäßwandbewegung und einer weiteren Ultraschall-Sende- und -Empfangseinrichtung (1, 4) mit nachgeschalteter weiterer Signalverarbeitungs-Schaltungsanordnung (9, 14, 16, 24—28) zum Ermitteln der Blutströmungsgeschwindigkeit sowie einer beiden Ultraschall-Sende- und -Empfangseinrichtungen (1, 4 bzw. 2, 5) und deren Signalverarbeitungs-Schaltungsanordnungen (9, 14, 16, 24—28 bzw. 9, 15, 17, 18, 20) nachgeschalteten Signalkorrelierungseinrichtung (22), dadurch gekennzeichnet, daß die Signalkorrelierungseineinrichtung eine Vergleichseinrichtung (22) zum Feststellen einer zeitlichen Koinzidenz von Ausgangssignalen der beiden Ultraschall-Sende- und -Empfangseinrichtungen (1, 4 bzw. 2, 5) mit dem Zweck einer Unterscheidung zwischen Systolen und ventrikulären Extrasystolen ist.

2. Kardiotachometer nach Anspruch 1, dadurch gekennzeichnet, daß der Ultraschallsender (1) der Ultraschall-Sende- und -Empfangseinrichtung (1, 4) zum Ermitteln der Blutströmungsgeschwindigkeit auf eine Ultraschallfrequenz im Bereich von 5 bis 10 MHz, vorzugsweise auf 5 MHz, abgestimmt ist, während der Ultraschallsender (2) der Ultraschall-Sende- und -Empfangseinrichtung (2, 5) zum Ermitteln der Gefäßwandbewegung auf eine Ultraschallfrequenz im Bereich von 1 bis 3 MHz, vorzugsweise auf 1 MHz, abgestimmt ist.

3. Kardiotachometer nach Anspruch 1 oder 2, dadurch gekennzeichnet, daß die Signalverarbeitungs-Schaltungsanordnung (9, 14, 16, 24–28), welche der Ultraschall-Sende- und -Empfangseinrichtung (1, 4) zum Ermitteln der Blutströmungsgeschwindigkeit nachgeschaltet ist, eine auf die von letzterer aufgenommenen Ultraschall-Dopplerfrequenzsignale unter Erzeugung von je einem Impuls für jede Pulswelle und für jede Verschiebungswelle in einem Blutgefäß ansprechende erste Impulserzeugungseinrichtung, insbesondere eine Rechteckimpulserzeugungseinrichtung, ist; und daß die Signalverarbeitungs-Schaltungsanordnung (9, 15, 17, 18, 20), welche der Ultraschall-Sende- und -Empfangseinrichtung (2, 5) zum Feststellen einer Gefäßwandbewegung nachgeschaltet ist, eine auf die von letzterer aufgenommenen Ultraschall-Dopplerfrequenzsignale unter Erzeugung von je einem Impuls für jede durch eine Pulswelle in einem Blutgefäß hervorgerufene Gefäßwandbewegung ansprechende zweite Impulserzeugungseinrichtung, insbesondere eine Rechteckimpulserzeugungseinrichtung, ist.

4. Kardiotachometer nach Anspruch 3, dadurch gekennzeichnet, daß jede der beiden Impulserzeugungseinrichtungen hintereinandergeschaltet einen Demodulator (9), einen Verstärker (14, 15) und/oder einen Regelverstärker (16, 17), einen Gleichrichter (18, 26) und eine Schwellwertdetektor- und Impulsformereinheit (20, 27) umfaßt.

5. Kardiotachometer nach Anspruch 3 oder 4, dadurch gekennzeichnet, daß die erste Impulserzeugungseinrichtung ein Bandpaßfilter (24) und ein Tiefpaßfilter (25) umfaßt, die parallel geschaltet sind und von denen das Bandpaßfilter (24) vorzugsweise einen Durchlaßbereich von 700 bis 1800 Hz und das Tiefpaßfilter (25) vorzugsweise einen Durchlaßbereich von 0 bis 500 Hz hat.

6. Kardiotachometer nach Anspruch 3, 4 oder 5, dadurch gekennzeichnet, daß die erste Impulserzeugungseinrichtung einen Impulsverlängerer (28) zum Ausschalten von durch eine Dicrotie in der Pulswelle verursachten gesonderten Impulsen aufweist, deren Impulsverlängerungsdauer, auf welche hindurchgehende Impulse verlängert werden, vorzugsweise etwa 300 msec beträgt.

7. Kardiotachometer nach einem der Ansprüche 1 bis 6, dadurch gekennzeichnet, daß eine digitale Signalverarbeitungseinheit (32) vorgesehen ist, die einen ersten Eingang (31) besitzt, welcher, vorzugsweise über eine Differenzierstufe (30), mit dem Ausgang (29) der Vergleichsstufe (22) verbunden ist.

8. Kardiotachometer nach Anspruch 7, dadurch gekennzeichnet, daß die digitale Signalverarbeitungseinheit (32) über einen zweiten Eingang (34) besitzt, welcher, vorzugsweise über eine Differenzierstufe (33), mit dem Ausgang der ersten Impulserzeugungseinrichtung, insbesondere mit dem Ausgang des Impulsverlängerers (28) verbunden ist.

9. Kardiotachometer nach Anspruch 7 oder 8, dadurch gekennzeichnet, daß die digitale Signalverarbeitungseinheit (32) einen dritten Eingang (36) hat, der mit dem Ausgang (40) eines Asystoliedetektors (35) verbunden ist, welcher seinerseits an den Ausgang der ersten Impulserzeugungseinrichtung, insbesondere an den Ausgang des Impulsverlängerers (28), angeschlossen ist.

10. Kardiotachometer nach einem der Ansprüche 7, 8 oder 9, dadurch gekennzeichnet, daß an die digitale Signalverarbeitungseinheit (32) eine oder mehrere Anzeigeeinrichtungen (41 bis 43) zum Anzeigen der Systolen- und/oder der Extrasystolen- und/oder der Systolen-plus-Extrasystolen-Häufigkeit aufweist sowie eine oder mehrere akustische und/oder optische Alarmanzeigeeinrichtungen (44 bis 48) zum Abgeben je eines Alarmsignals, wenn die Systolenhäufigkeit außerhalb eines vorgebbaren Bereiches liegt und/oder wenn die Häufigkeit der Extrasystolen und/oder der Systolen-plus-Extrasystolen einen vorbestimmten Wert überschreitet und/oder wenn während einer vorbestimmten Zeitdauer weder eine Systole noch eine Extrasystole auftritt.

11. Kardiotachometer nach einem der Ansprüche 1 bis 10, dadurch gekennzeichnet, daß die beiden Ultraschall-Sende- und -Empfangseinrichtungen (1, 4; 2, 5) in einem Meßkopf (55) zusammengefaßt sind, wobei die Ultraschallsender (1, 2) in der Mitte einer parallel zu einer Ankopplungsmembran für die Ankopplung an die Körperoberfläche des Benutzers verlaufenden Querschnittsfläche des Meßkopfes (55) angeordnet sind, während die Ultraschallempfänger (4, 5) je in mehrere, vorzugsweise mindestens drei, miteinander parallelgeschaltete einzelne, gegenüber den Ultraschallsender (1, 2) relativ kleinflächige Ultraschallempfänger (401 bis 403; 501 bis 503) aufgeteilt sind, die, vorzugsweise symmetrisch, um die Ultraschallsender (1, 2) herum angeordnet sind.

12. Kardiotachometer nach Anspruch 11, dadurch gekennzeichnet, daß der Ultraschallsender (1) für die Ermittlung der Strömungsgeschwindigkeit einen Ultraschall-Abstrahlkegel (60) mit einem Öffnungswinkel ($\alpha$) von wesent-

lich mehr als 90°, vorzugsweise von etwa 120°, hat.

13. Kardiotachometer nach Anspruch 11 oder 12, dadurch gekennzeichnet, daß der Ultraschallsender (2) für die Feststellung der Gefäßwandbewegung einen Ultraschall-Abstrahlkegel (61) mit einem Öffnungswinkel ($\beta$) von wesentlich weniger als 90°, vorzugsweise von etwa 60°, hat.

14. Kardiotachometer nach Anspruch 11, 12 oder 13, dadurch gekennzeichnet, daß die Ultraschallempfänger (401 bis 403) für die Ermittlung der Strömungsgeschwindigkeit unter einem Neigungswinkel von 15 bis 30° und die Ultraschallempfänger (501 bis 503) unter einem Neigungswinkel von 30 bis 45° ihrer Haupt-Schallempfangsrichtung zur Körperoberfläche angeordnet sind.

15. Kardiotachometer nach einem der Ansprüche 1 bis 14, dadurch gekennzeichnet, daß die Stromversorgung für die Ultraschallempfänger (4, 5) und einen Teil der beiden Signalverarbeitungs-Schaltungsanordnungen (9, 15, 17, 18, 20) über eine periodisch ein- und ausschaltende Takteinheit (53) geschaltet ist.

**Claims**

1. A cardiotachometer for determining the heart rate under consideration from ventricular extra-systoles by means of an ultrasonic transmitting and receiving device (2, 5) which operates according to the Doppler ultrasound principle, having a signal processing-circuit arrangement (9, 15, 17, 18, 20) connected downstream, to determine a vessel wall movement, and another ultrasonic transmitting and receiving device (1, 4) having another signal processing-circuit arrangement (9, 14, 16, 24—28) connected downstream, to determine the blood flow rate, and having a signal correlation device (22) connected downstream of the two ultrasonic transmitting and receiving devices (1, 4 and 2, 5 respectively) and of the signal processing-circuit arrangements thereof (9, 14, 16, 24—28 and 9, 15, 17, 18, 20 respectively), characterised in that the signal correlation device is a comparator (22) for establishing a time coincidence between output signals of the two ultrasonic transmitting and receiving devices (1, 4 and 2, 5 respectively) with the objective of a differentiation between systoles and ventricular extra-systoles.

2. A cardiotachometer according to claim 1, characterised in that the ultrasonic transmitter (1) of the ultrasonic transmitting and receiving device (1, 4) for determining the blood flow rate is coordinated with an ultrasonic frequency ranging from 5 to 10 MHz, preferably 5 MHz, while the ultrasonic transmitter (2) of the ultrasonic transmitting and receiving device (2, 5) for determining the vessel wall movement is coordinated with an ultrasonic frequency ranging from 1 to 3 MHz, preferably 1 MHz.

3. A cardiotachometer according to claim 1 or 2, characterised in that the signal processing-circuit arrangement (9, 14, 16, 24—28) which is connected downstream of the ultrasonic transmitting and receiving device (1, 4) for determining the blood flow rate is a first pulse generating device, in particular a rectangular pulse generating device responding to the ultrasonic Doppler frequency signals picked up by the latter, with the production of each one pulse for each pulse wave and for each displacement wave in a blood vessel; and the signal processing-circuit arrangement (9, 15, 17, 18, 20) which is connected downstream of the ultrasonic transmitting and receiving device (2, 5) for determining a vessel wall movement is a second pulse generating device, in particular a rectangular pulse generating device responding to the ultrasonic Doppler frequency signals picked up by the latter, with the production of each one pulse for each vessel wall movement caused by a pulse wave in a blood vessel.

4. A cardiotachometer according to claim 3, characterised in that each of the two pulse generating devices comprises, connected in tandem, a demodulator (9), an amplifier (14, 15) and/or a servo amplifier (16, 17), a rectifier (18, 26) and a threshold value detector and pulse shaper unit (20, 27).

5. A cardiotachometer according to claim 3 or 4, characterised in that the first pulse generating device comprises a band-pass filter (24) and a low-pass filter (25) which are connected in parallel, and of which the band-pass filter (24) preferably has a pass-range of from 700 to 1800 Hz, and the low-pass filter (25) preferably has a pass-range of from 0 to 500 Hz.

6. A cardiotachometer according to claim 3, 4 or 5, characterised in that the first pulse generating device has a pulse extender (28) to eliminate separate pulses caused by dicrolic double pulse in the pulse wave, the pulse extension duration of these pulses, to which continuous pulses are extended, preferably amounts to about 300 msec.

7. A cardiotachometer according to one of claims 1 to 6, characterised in that a digital signal processing unit (32) is provided which has a first input (31) which is connected to the output (29) of the comparison stage (22), preferably via a differentiation stage (30).

8. A cardiotachometer according to claim 7, characterised in that the digital signal processing unit (32) has a second input (34) which is connected to the output of the first pulse generating device, in particular to the output of the pulse extender (28), preferably via a differentiation stage (33).

9. A cardiotachometer according to claim 7 or 8, characterised in that the digital signal processing unit (32) has a third input (36) which is connected to the output (40) of an asystole detector (35) which, for its part, is connected to the output of the first pulse generating device, in particular to the output of the pulse extender (28).

10. A cardiotachometer according to one of claims 7, 8 or 9, characterised in that to the digital signal processing unit (32) has one or more indicators (41 to 32) to indicate the systole- and/or the extra-systole- and/or the systole-plus-extra-systole rate, and one or more acoustic and/or optical alarm indicators (44 to 48) to each emit an alarm signal if the systole rate lies outside a pre-determinable range, and/or if the rate of the extra-systoles and/or of the systole-plus-extra-systoles exceeds a pre-determined value, and/or if neither a systole nor an extra-systole occurs during a pre-determined period of time.

11. A cardiotachometer according to one of claims 1 to 10, characterised in that the two ultrasonic transmitting and receiving devices (1, 4; 2, 5) are combined in a measuring head (55), the ultrasonic transmitters (1, 2) being arranged in the middle of a cross sectional surface of the measuring head (55) which extends parallel to a coupling membrane for coupling to the body surface of the user, while the ultrasonic receivers (4, 5) are each divided into several, preferably at least three, parallel interconnected, individual ultrasonic receivers (401 to 403; 501 to 503) which have a relatively small surface compared to the ultrasonic transmitters (1, 2), which ultrasonic receivers (401 to 403; 501 to 503) are positioned around the ultrasonic transmitters (1, 2), preferably in a symmetric arrangement.

12. A cardiotachometer according to claim 11, characterised in that the ultrasonic transmitter (1) for determining the flow rate has an ultrasonic radiation cone (60) having an aperture angle ($\alpha$) of substantially more than 90°, preferably about 120°.

13. A cardiotachometer according to claim 11 or 12, characterised in that the ultrasonic transmitter (2) for determining the vessel wall movement has an ultrasonic radiation cone (61) having an aperture angle ($\beta$) of substantially less than 90°, preferably about 60°.

14. A cardiotachometer according to claim 11, 12 or 13, characterised in that the ultrasonic receivers (401 to 403) for determining the flow rate are positioned at an angle of incline of from 15 to 30°, and the ultrasonic receivers (501 to 503) are positioned at an angle of incline of from 30 to 45° of their main sound receiving direction to the surface of the body.

15. A cardiotachometer according to one of claims 1 to 14, characterised in that the power supply for the ultrasonic receivers (4, 5) and for a part of the two signal processing-circuit arrangements (9, 15, 17, 18, 20) is connected via a clock unit (53) which switches on and off periodically.

**Revendications**

1. Cardiotachymètre pour déterminer la fréquence cardiaque en tenant compte des extrasystoles ventriculaires, à l'aide d'un dispositif d'émission et de réception d'ultrasons (2, 5) fonctionnant selon le principe DOPPLER par ultrasons à la suite duquel est branchée une installation de traitement de signaux (9, 15, 17, 18, 20) pour déterminer un mouvement de paroi de vaisseau, et un autre dispositif d'émission et de réception d'ultrasons (1, 4) à la suite duquel est branchée une autre installation de traitement de signaux (9, 14, 16, 24—28) pour déterminer la vitesse de circulation du sang, ainsi que d'un dispositif de corrélation de signaux (22) branché à la suite de deux dispositifs d'émission et de réception d'ultrasons (1, 4 resp. 2, 5) et de leurs installations de traitement de signaux (9, 14, 16, 24—28 resp. 9, 15, 17, 18, 20), caractérisé en ce que le dispositif de corrélation de signaux est une installation de comparaison (22) pour déterminer une coïncidence temporelle des signaux de sortie des deux installations d'émission et de réception d'ultrasons (1, 4 resp. 2, 5) dans le but de distinguer entre des systoles et des extrasystoles ventriculaires.

2. Cardiotachymètre selon la revendication 1, caractérisé en ce que l'émetteur d'ultrasons (1) du dispositif d'émission et de réception d'ultrasons (1, 4) pour obtenir la vitesse de circulation du sang est accordé sur une fréquence d'ultrasons dans la zone de 5 à 10 MHz, de préférence sur 5 MHz, alors que l'émetteur d'ultrasons (2) du dispositif d'émission et de réception d'ultrasons (2, 5) pour obtenir le mouvement de paroi du vaisseau est accordé sur une fréquence d'ultrasons dans la zone de 1 à 3 MHz, de préférence sur 1 MHz.

3. Cardiotachymètre selon la revendication 1 ou 2, caractérisé en ce que l'installation de traitement de signaux (9, 14, 16, 24—28), qui est branchée à la suite du dispositif d'émission et de réception d'ultrasons (1, 4) pour déterminer la vitesse de circulation du sang, est une première installation de génération de signaux, en particulier une installation de génération de signaux rectangulaires, réagissant aux signaux de fréquence DOPPLER d'ultrasons prélevés sur ce dispositif pour générer une impulsion pour chaque onde de pulsion et pour chaque onde de translation, et en ce que l'installation de traitement de signaux (9, 15, 17, 18, 20), qui est branchée à la suite du dispositif d'émission et de réception d'ultrasons (2, 5) pour déterminer un mouvement de paroi de vaisseau, est une deuxième installation de génération de signaux, en particulier de signaux rectangulaires, réagissant aux signaux de fréquence DOPPLER d'ultrasons prélevés sur ce dernier dispositif pour générer une impulsion pour chaque mouvement de paroi de vaisseau provoqué par une onde de pulsion dans un vaisseau sanguin.

4. Cardiotachymètre selon la revendication 3, caractérisé en ce que chacune des deux installations de génération d'impulsions comprend en série un démodulateur (9), un amplificateur (14, 15) et/ou un amplificateur de régulation (16, 17), un redresseur (18, 26) et une unité de détection de valeur de seuil et de mise en forme d'impulsions (20, 27).

5. Cardiotachymètre selon la revendication 3 ou 4, caractérisé en ce que la première installation de génération d'impulsions comprend un filtre passe-bande (24) et un filtre passe-bas (25), qui sont branchés en parallèle, le filtre passe-bande (24) ayant de préférence une bande passante de 700 à 1800 Hz et le filtre passe-bas (25) ayant de préférence une bande passante de 0 à 500 Hz.

6. Cardiotachymètre selon la revendication 3, 4 ou 5, caractérisé en ce que la première installation de génération d'impulsions présente un allongeur d'impulsions (28) pour éliminer des impulsions particulières dues à une dicrotie dans l'onde de pulsion, dont la durée d'allongement d'impulsion, selon laquelle les impulsions traversantes sont allongées, est de préférence d'environ 300 msec.

7. Cardiotachymètre selon l'une des revendications 1 à 6, caractérisé en ce qu'il est prévu une unité de traitement de signaux numérique (32) qui possède une première entrée (31) qui est reliée, de préférence par l'intermédiaire d'un étage de différentiation (30), avec la sortie (29) de l'étage de comparaison (22).

8. Cardiotachymètre selon la revendication 7, caractérisé en ce que l'unité de traitement de signaux numérique (32) possède une deuxième entrée (34) qui est reliée, de préférence par l'intermédiaire d'un étage de différentiation (33), avec la sortie de la première installation de génération d'impulsions, en particulier avec la sortie de l'allongeur d'impulsions (28).

9. Cardiotachymètre selon la revendication 7 ou 8, caractérisé en ce que l'unité de traitement de signal numérique (32) a une troisième entrée (36) qui est reliée à la sortie (40) d'un détecteur d'asystolie (35), qui est raccordé de son côté à la sortie de la première installation de génération d'impulsions, en particulier à la sortie de l'allongeur d'impulsions (28).

10. Cardiotachymètre selon l'une des revendications 7, 8 ou 9, caractérisé en ce que l'unité de traitement de signaux numérique (32) possède un ou plusieurs dispositifs d'affichage (41 à 43) pour afficher la fréquence des systoles et/ou des extrasystoles et/ou des systoles plus des extrasystoles, ainsi qu'un ou plusieurs dispositifs avertisseurs d'alarme acoustiques et/ou optiques (44 à 48) pour délivrer chaque fois un signal d'alarme quand la fréquence des systoles se trouve en dehors d'une zone préétablie et/ou quand la fréquence des extrasystoles et/ou des systoles plus des extrasystoles dépasse une valeur prédéterminée et/ou quand ni une systole ni une extrasystole n'apparaît pendant une période prédéterminée.

11. Cardiotachymètre selon l'une des revendications 1 à 10, caractérisé en ce que les deux dispositifs d'émission et de réception d'ultrasons (1, 4, 2, 5) sont rassemblés dans une tête de mesure (55), les émetteurs d'ultrasons (1, 2) étant disposés au milieu d'une surface transversale de la tête de mesure (55), qui s'étend parallèlement à une membrane de couplage pour le couplage à la surface du corps de l'utilisateur, tandis que les récepteurs d'ultrasons (4, 5) sont subdivisés en plusieurs, de préférence au moins trois, récepteurs d'ultrasons (401 à 403; 501 à 503) différents, de surface relativement petite par rapport aux émetteurs d'ultrasons (1, 2) et branchés en parallèle entre eux et qui sont disposés autour des émetteurs d'ultrasons (1, 2) de préférence de façon symétrique.

12. Cardiotachymètre selon la revendication 11, caractérisé en ce que l'émetteur d'ultrasons (1) pour la détermination de la vitesse de circulation a un cône d'émission d'ultrasons (60) présentant un angle d'ouverture ($\alpha$) sensiblement supérieur à 90°, de préférence égal à 120° environ.

13. Cardiotachymètre selon la revendication 11 ou 12, caractérisé en ce que l'émetteur d'ultrasons (2) pour la détermination du mouvement d'une paroi de vaisseau a un cône d'émission d'ultrasons (61) présentant un angle d'ouverture ($\alpha$) sensiblement inférieur à 90°, de préférence égal à environ 60°.

14. Cardiotachymètre selon la revendications 11, 12 ou 13, caractérisé en ce que les récepteurs d'ultrasons (401 à 403) pour la détermination de la vitesse de circulation sont disposés suivant un angle d'inclinaison de 15 à 30° et les récepteurs d'ultrasons (501 à 503) sont disposés suivant un angle d'inclinaison de 30 à 45° de leur direction principale de réception d'ultrasons par rapport à la surface du corps.

15. Cardiotachymètre selon l'une des revendications 1 à 14, caractérisé en ce que l'alimentation en courant pour les récepteurs d'ultrasons (4, 5) et une partie des deux installations de traitements de signaux (9, 15, 17, 18, 20) est connectée par l'intermédiaire d'une unité d'horloge (53) se branchant et se débranchant périodiquement.

HF-Oszillatoreinheit
HF-Empfängereinheit
Memodulatoreinheit
Verstärker
Verstärker
Regelverstärker
Regelverstärker
Tiefpaßf.
Bandpaßf.
Gleichrichter
Gleichrichter
Schwellw.-Impulsf.
Schwellwertdetektor u. Impulsformer
Impulsverläng.
Systolen-Zählimpulse
Diff.
Vergleichsstufe
Systolen-u. Extrasyst.-Zählimp.
Diff.-Stufe
Asystolie-signal
Asystolie-Detektor
Taktgeber
DIGITALE SIGNALVERARBEITUNGS-EINHEIT
110
4
114
SY
ESY
S+E
ASY

FIG. 1

HF-Oszillatoreinheit
Takteinheit
Verstärker
Verstärker
FIG. 2
HF-Empfängereinheit
Spannungsverdoppler
DIGITALE SIGNALVERARBEITUNGS-EINHEIT
1
2
3
53
14
15
54
6
4
5
50
49
51
52
32

EKG
Systole
S
Extrasystole
ES
FIG. 3
I
Pulswelle
A
A'
DE
B
Verschiebungswelle
II
Gefäßwandbewegung
F
III
C
C'
D
IV
G
V
E
H
VI
J
J'
VII
K
L
K'
L'
VIII

FIG.4
55
401
503
502
403
402
2
1
501

FIG.5
64
2
1
59
403
502
56
55
58
57
γ
62
61
β
δ
60
α
63

FIG. 6

64
403
2
1
502
Epoxy
55
58
15°
65
5-15 mm
66